# EUROPEAN PATENT SPECIFICATION

(11) **EP 0 682 650 B1**
(45) Date of publication and mention of the grant of the patent: **20.05.1998**
(21) Application number: 94906916.5
(22) Date of filing: 03.02.1994
(51) Int. Cl.: C07C 45/58, C07C 29/36

(54) **PROCESS FOR MAKING 1,3-DIOLS AND 3-HYDROXYALDEHYDES**
VERFAHREN ZUR HERSTELLUNG VON 1,3-DIOLEN UND 3-HYDROXYALDEHYDEN
PROCEDE DE PRODUCTION DE 1,3 DIOLS ET DE 3-HYDROXYALDEHYDES

(30) Priority: 05.02.1993 US 13834; 05.02.1993 US 13835; 05.02.1993 US 13836; 13.07.1993 US 91107; 13.07.1993 US 91108; 01.10.1993 US 130258; 01.10.1993 US 130260
(43) Date of publication of application: 22.11.1995
(73) Proprietor: SHELL INTERNATIONALE RESEARCH MAATSCHAPPIJ B.V., 2596 HR Den Haag (NL)
(72) Inventor: SLAUGH, Lynn, Henry, Houston, TX 77070 (US); WEIDER, Paul, Richard, Houston, TX 77083 (US); ARHANCET, Juan, Pedro, Katy, TX 77450 (US); LIN, Jiang-Jen, Houston, TX 77083 (US)
(86) International application number: EP9400346
(87) International publication number: WO9418149

(56) References cited:
- US-A- 3 456 017
- US-A- 3 463 819
- US-A- 3 527 818
- US-A- 5 256 827

## Description

This invention relates to a process for making 1,3-diols and 3-hydroxyaldehydes by hydroformylating 1,2-epoxides using selected ditertiary phosphine-modified cobalt carbonyl catalysts.

3-Hydroxyaldehydes are useful chemical intermediates. They can be readily converted to 1,3-diols which are useful as antifreese agent (1,3-propanediol) and as a chemical intermediates in the formation of polyethers, polyesters, polyoxyalkalene glycols which find use in fibers, additives, stabilizers and the like.

US Patents Nos. 3,463,819 and 3,456,017 teach a process for the hydroformylation of epoxides to produce 3-hydroxyaldehydes and 1,3-diols using phosphine-modified cobalt carbonyl catalysts. These references use a large amount of catalyst compared to the starting epoxide amounts used. The use of large amounts of catalyst is expensive and can make a commercial process uneconomical. The hydroformylation of epoxides of higher carbon number than ethylene oxide produces lower selectivities and yields of product when compared to the hydroformylation of ethylene oxide.

US Patent No. 3,687,981 uses dicobalt octacarbonyl as a catalyst and discloses hydroquinone as a catalyst stabilizer in the hydroformylation of ethylene oxide. Inorganic halogen-containing compounds, such as hydrochloric acid, are disclosed hydroformylation promoters, i.e., compounds that increase the conversion of ethylene oxide to the desired product. Trace amounts are said to be useful.

In US Patents Nos. 3,401,204 and 3,527,818 ditertiary phosphine ligands and cobalt catalysts prepared therefrom are described as being suitable for hydroformylating olefins to alcohols.

It is an object of this invention to use an improved catalyst system comprising cobalt-ditertiary phosphine ligand catalyst to hydroformylate epoxides having a least 2 carbon atoms to the corresponding 3-hydroxyaldehyde and 1,3-diol products in a high yield. Accordingly, this invention provides a process for making 1,3-diols and 3-hydroxyaldehydes by hydroformylating 1,2-epoxides which process comprises intimately contacting
(a) a 1,2-epoxide having at least 2 carbon atoms,
(b) ditertiary phosphine-modified cobalt carbonyl catalyst, said phosphine comprising a hydrocarbylene-bis(monophosphabicycloalkane) in which each phosphorus atom is joined to hydrocarbylene and is a member of a bridge linkage without being a bridgehead atom and which hydrocarbylene-bis(monophosphabicycloalkane) has 11 to 300, preferably 11 to 200, more preferably 11 to 100 and most preferably 18 to 80 carbon atoms, of which 5 to 12, preferably 6 to 12, more preferably 7 to 12 and most preferably 8 carbon atoms together with a phosphorus atom are members of each of the two bicyclic skeletal structures,
(c) carbon monoxide, and
(d) hydrogen, the molar ratio of carbon monoxide to hydrogen being from 4:1 to 1:6, preferably from 1:1 to 1:4
in liquid-phase solution in an inert reaction solvent, at a temperature of from 30 to 150°C and a pressure of from 345 to 68948 kPa (50 to 10,000 psi).

1,2-Epoxides are hydroformylated by reaction with carbon monoxide and hydrogen in the presence of a catalyst system comprising a ditertiary phosphine-modified cobalt carbonyl catalyst. The reaction products comprise primarily 3-hydroxyaldehydes (and oligomers thereof) and 1,3-diols. The ratio of the two products can be adjusted by adjusting the amounts of catalysts present in the reaction mixture, the reaction temperature and/or the amount of hydrogen present in the reaction mixture. When the term "3-hydroxyaldehyde" is used herein is understood to mean the monomer as well as dimers, trimers and higher oligomers of the 3-hydroxyaldehyde. The 3-hydroxyaldehyde and/or 1,3-diol product will have one more carbon atom that the reactant epoxide. In a preferred embodiment, lower amounts of catalyst are used to produce primarily the aldehyde and its oligomers which are then hydrogenated to the 1,3-diol in a separate hydrogenation step using conventional hydrogenation catalyst and hydrogen. The use of the particular ditertiary phosphines as complexing ligands for the cobalt catalyst results in catalysts providing very high yields of hydroformylated products, higher than that provided by the use of conventional phosphine ligands.

The epoxide reactant comprises an organic compound, two carbons of which are connected by an oxy linkage as well as by a carbon-carbon single bond. The preferred compounds are those having the oxy linkage at the 1,2-position. In general terms the compounds comprise hydrocarbyl-epoxides, having carbon numbers of at least 2, preferably having carbon numbers ranging from 2 to 30, more preferably from 2 to 20, and most preferably from 2 to 10. The hydrocarbyl moiety may be any nonacetylenic acyclic or cyclic organic radical. Wide variation is possible in that the (nonacetylenic) acyclic or cyclic hydrocarbyl group may be aryl, alkyl, alkenyl, aralkyl, cycloalkyl, straight chain, branched chain, large or small. Preferred compounds are 1,2-epoxyalkanes and 1,2-epoxyalkenes. Suitable examples of 1,2-epoxyalkanes include ethylene oxide, propylene oxide, isobutylene oxide, 1,2-epoxypentane, 1,2-epoxy-4-methylpentane, 1,2-epoxyoctane, 3-cyclohexyl-1,2-epoxypropane, 1,2-epoxy-2,2,4-trimethylhexane, 1,2-epoxydecane and 1,2-epoxydodecane. Suitable examples of 1,2-epoxyalkenes include 1,2-epoxypent-4-ene, 1,2-epoxyhex-5-ene, 1,2-epoxy-4-methylhex-5-ene, 1,2-epoxyoct-5-ene, 1,2-epoxydec-9-ene and 1,2-epoxydodec-11-ene. Ethylene oxide and propylene oxide are most preferred.

The process is conducted, in one modification, by charging the epoxide reactant, catalysts, optional catalyst promoter(s) and reaction solvent to an autoclave or similar pressure reactor and introducing the hydrogen and carbon monoxide while the reaction mixture is maintained at reaction temperature. Alternatively, the process is conducted in a continuous manner as by contacting the reactants, catalysts and optional catalyst promoter(s) during passage through a reactor which is typically tubular in form. For best results the process is conducted under conditions of elevated temperature and pressure. Reaction temperatures range from 30 to 150°C, preferably from 50 to 125°C, and most preferably from 70 to 110°c. The reaction pressure is desirably in the range of from 345 to 68948 kPa (50 to 10,000 psi), preferably from 3447 to 20684 kPa (500 to 3000 psi). In one modification of the process, inert gaseous diluent is present, e.g., inert gaseous diluents such as argon, helium, methane, and nitrogen, in which case the reaction pressure is properly considered to be the sum of the partial pressures of the materials other than the diluent. In the preferred modification of the process, however, the reaction is conducted in the substantial absence of added diluent.

The course of the reaction is easily followed by observing the pressure decrease within the reactor, by in situ infrared absorption techniques or by periodic withdrawal and analysis of samples from the reaction system. At the conclusion of reaction, the product mixture is separated by conventional methods such as selective extraction, fractional distillation, decantation, selective crystallization and the like. The unreacted starting material as well as the catalyst and reaction solvent are suitably recycled for further reaction.

The catalysts employed in the process of the invention are ditertiary phosphine-modified cobalt carbonyl complexes. Particularly preferred ditertiary phosphines are chosen from α,Ω-hydrocarbylene-P,P'-bis(monophosphabicyclononanes) in which ring systems (a) each phosphorus atom is a member of a bridge linkage, (b) each phosphorus atom is not in a bridgehead position, and (c) each phosphorus atom is not a member of the bicyclic system of the other, and (d) the smallest phosphorus-containing rings contain at least four, preferably at least five atoms. In addition to the hydrocarbylene substitution on the phosphorus atoms, the ring carbons may also be substituted. The hydrocarbylene is preferably selected from ethylene, propylene and butylene. Most preferably the hydrocarbylene is ethylene and each of the monophosphabicyclononane moieties of the ditertiary phosphine is independently selected from 9-phosphabicyclo[4.2.1]-nonane and 9-phosphabicyclo[3.3.1]nonane. As used herein the term "9-phosphabicyclononyl" or "9-phosphabicyclononane" will refer to phosphabicyclo[4.2.1]nonane and 9-phosphabicyclo[3.3.1]nonane moieties and mixtures thereof.

In general terms the ditertiary phosphine ligands used to form the cobalt-carbonyl-phosphine complexes comprise bicyclic heterocyclic ditertiary phosphines. One class of such compounds has from 11 to 300, preferably 11 to 200, more preferably 11 to 100 and most preferably 18 to 80 carbon atoms, and is represented by the formula 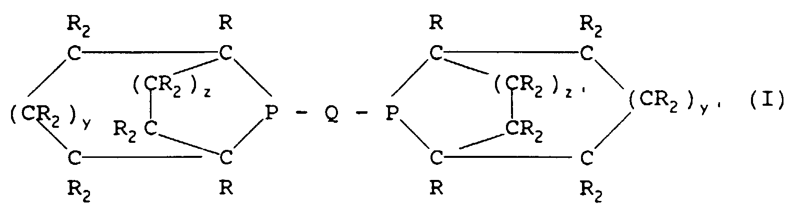 where Q represents hydrocarbylene of up to 30 carbon atoms; R independently represents hydrogen and hydrocarbyl of 1 to 30 carbon atoms; y and z represent zero or positive integers whose sum is from 0 to 7; y' and z', independent of the values of y and z, represent zero or positive integers whose sum is from 0 to 7; preferably y and z represent positive integers whose sum is from 1 to 7, more preferably from 2 to 7 and most preferably 3 with each of which having a minimum value of 1; y' and z', independent of the values of y and z, represent positive integers whose sum is from 1 to 7, more preferably from 2 to 7 and most preferably 3 with each of which having a minimum value of 1. It is to be understood that in the foregoing graphic formula and those appearing hereinafter the line portion of the structure represents a conventional organic chemical covalent bond with saturated carbon atom at each indicated intersection, the saturation being by the required number of hydrogen atoms or lower alkyl radicals.

Hence, a preferred group of bicyclic heterocyclic ditertiary phosphines includes those represented by Formula I where Q represents hydrocarbylene of 2 to 30 carbons and especially of 2 to 20; y and z represent positive integers whose sum is 3 and each of which has a minimum value of 1; y' and z', independent of the values of y and z, represent positive integers whose sum is 3 and each of which has a minimum value of 1; and R represents hydrogen and optionally hydrocarbyl of from 1 to 20 carbons.

The term "hydrocarbylene" is used in its accepted meaning as representing a diradical formed by removal of two hydrogen atoms from a carbon atom or preferably one hydrogen atom from each of two different carbon atoms of a hydrocarbon. The hydrocarbylene groups represented by Q in the formula above may be any nonacetylenic acyclic or cyclic organic radical composed solely of carbon and hydrogen. Wide variation is possible in that the (nonacetylenic) acyclic or cyclic hydrocarbylene group may be arene, alkylene, alkenylene, aralkylene, cycloalkylene, straight chain, branched chain, large or small. Representative hydrocarbylene groups include methylene, ethylene, trimethylene, tetramethylene, butylene, pentamethylene, pentylene, methylpentylene, hexamethylene, hexenylene, ethylhexylene, dimethylhexylene, octamethylene, octenylene, cyclooctylene, methylcyclooctylene, dimethylcyclooctylene, isooctylene, dodecamethylene, hexadecenylene, octadecamethylene, eicosamethylene, hexacosamethylene, triacontamethylene, and phenylenediethylene. A particularly useful class of bicyclic heterocyclic ditertiary phosphines is that containing only carbon, hydrogen, and phosphorus atoms. Substituted hydrocarbylene groups are also contemplated and may contain a functional group such as the carbonyl, carboxyl, nitro, amino, hydroxy (e.g. hydroxyethyl), cyano, sulfonyl, and sulfoxyl groups. A particularly useful group of ditertiary phosphines consists of those in which Q is hydrocarbylene of up to 30 carbon atoms, preferably of from 2 to 30 carbon atoms; more preferably from 2 to 20 carbons, even more preferably from 2 to 10. In a preferred embodiment Q is ethylene, propylene or butylene, more preferably ethylene.

The term "hydrocarbyl" is used in its accepted meaning as representing a radical formed by removal of one hydrogen atom from a carbon atom of a hydrocarbon. The hydrocarbyl groups represented by R in the formula above may be any nonacetylenic acyclic or cyclic organic radical composed solely of carbon and hydrogen. Wide variation is possible in that the (nonacetylenic) acyclic or cyclic hydrocarbyl group may be aryl, alkyl, alkenyl, aralkyl, cycloalkyl, straight chain, branched chain, large or small. Representative hydrocarbyl groups include methyl, ethyl, butyl, pentyl, methylpentyl, hexenyl, ethylhexyl, dimethylhexyl, octamethyl, octenyl, cyclooctyl, methylcyclooctyl, dimethylcyclooctyl, isooctyl, dodecyl, hexadecenyl, octyl, eicosyl, hexacosyl, triacontyl, and phenylethyl. Substituted hydrocarbyl groups are also contemplated and may contain a functional group such as the carbonyl, carboxyl, nitro, amino, hydroxy (e.g. hydroxyethyl), cyano, sulfonyl, and sulfoxyl groups. Preferably R is hydrogen or hydrocarbyl, preferably alkyl, having 1 to 30, preferably 1 to 20 and most preferably from 8 to 20 carbon atoms.

Ditertiary phosphine ligands and cobalt catalysts prepared therefrom are known in the art and their method of preparation are described in detail in US Patents Nos. 3,401,204 and 3,527,818.

Generically, the ditertiary phosphine-modified cobalt complexes are characterized as dicobalt hexacarbonyl complexes of additionally present ditertiary phosphine ligand sufficient to provide one phosphorus complexing atom for each atom of cobalt present within the complexed molecule.

The phosphine ligands may also be partially oxidized to phosphine oxides in order to enhance the activity of the cobaltligand complex. The oxidation is carried out with an oxidant under mild oxidizing conditions such that an oxygen will bond to a phosphorus, but phosphorus-carbon, carbon-carbon and carbon-hydrogen bonds will not be disrupted. By suitable selection of temperatures, oxidants and oxidant concentrations such mild oxidation can occur. The oxidation of the phosphine ligands is carried out prior to the forming of the catalyst complex.

Suitable oxidizing agents include peroxy-compounds, persulfates, permanganates, perchromates and gaseous oxygen. Preferred compounds, for ease of control, are the peroxy-compounds. Peroxy-compounds are those which contain the peroxy (-O-O-) group. Suitable peroxy-compounds may be inorganic or organic. Suitable inorganic compounds include hydrogen peroxide as well as inorganic compounds which in contact with water liberate hydrogen peroxide, such compounds include the mono-valent, di-valent and trivalent metal peroxides as well as hydrogen peroxide addition compounds. Also suitable are the organic peroxy-compounds, including hydroperoxides; α-oxy- and α-peroxy- hydroperoxides and peroxides; peroxides; peroxyacids; diacyl peroxides; and peroxyesters. Suitable peroxyorgano-compounds include t-butyl hydroperoxide, cumene hydroperoxide, dibenzoyl peroxide and peroxyacetic acid. Peroxy-compounds suitable for carrying out the oxidation process are known in the art, and suitable examples can be found in The Encyclopedia of Chemical Technology, Vol. 17, pp. 1-89, Third Edition (John Wiley & Sons, 1982).

Typically oxidation is carried out by adding to the ligand a measured amount of oxidizing agent, sufficient to carry out the degree of oxidation required. The ligand may be dissolved in a suitable solvent. The oxidizing agent is typically added slowly over a period of time to control the oxidizing conditions. The temperature is maintained to provide mild oxidizing conditions. When hydrogen peroxide is used as the oxidizing agent, the temperature is typically maintained at room temperature.

The oxidation of the ligand is carried out to provide no more than 0.5 oxygen atoms per phosphorus atoms, on the average, in the oxidized ligand product. Preferably the ratio of oxygen atoms to phosphorus atoms in the oxidized ligand will range, on the average, from 0.01:1 to 0.5:1, and more preferably from 0.05:1 to 0.3:1.

The cobalt catalysts can be prepared by a diversity of methods. A convenient method is to combine a cobalt salt, organic or inorganic, with the desired phosphine ligand, for example, in liquid phase followed by reduction and carbonylation. Suitable cobalt salts comprise, for example, cobalt carboxylates such as acetates, octanoates, etc., which are preferred, as well as cobalt salts of mineral acids such as chlorides, fluorides, sulfates, sulfonates, etc. Operable also are mixtures of these cobalt salts. It is preferred, however, that when mixtures are used, at least one component of the mixture be cobalt alkanoate of 6 to 12 carbon atoms. The valence state of the cobalt may be reduced and the cobalt-containing complex formed by heating the solution in an atmosphere of hydrogen and carbon monoxide. The reduction may be performed prior to the use of the catalysts or it may be accomplished simultaneously with the hydroformylation process in the hydroformylation zone. Alternatively, the catalysts can be prepared from a carbon monoxide complex of cobalt. For example, it is possible to start with dicobalt octacarbonyl and, by heating this substance with a suitable phosphine ligand, the ligand replaces one or more, preferably at least two, of the carbon monoxide molecules, producing the desired catalyst. When this latter method is executed in a hydrocarbon solvent, the complex may be precipitated in crystalline form by cooling the hot hydrocarbon solution. This method is very convenient for regulating the number of carbon monoxide molecules and phosphine ligand molecules in the catalyst. Thus, by increasing the proportion of phosphine ligand added to the dicobalt octacarbonyl, more of the carbon monoxide molecules are replaced.

The optimum ratio of 1,2-epoxide feed to phosphine-modified cobalt carbonyl complex will in part depend upon the particular cobalt complex employed. However, molar ratios of 1,2-epoxide to cobalt complex from 2:1 to 10,000:1 are generally satisfactory, with molar ratios of from 50:1 to 500:1 being preferred. When batch processes are used, it is understood that the above ratios refer to the initial starting conditions. In one modification, the ditertiary phosphine-modified cobalt carbonyl complex is employed as a preformed material, being prepared as by reaction of a cobalt salt with carbon monoxide and hydrogen in the presence of the ditertiary. phosphine ligand, then isolated and subsequently utilized in the present process. In an alternate modification, the ditertiary phosphine-modified cobalt complex is prepared in situ as by addition to the reaction mixture of a cobalt salt or dicobalt octacarbonyl together with the ditertiary phosphine ligand whose introduction into the catalyst complex is desired.

In practice, it is preferable to employ the ditertiary phosphine-modified cobalt complex in conjunction with a minor proportion of excess ditertiary phosphine ligand which is the same as or is different from the ditertiary phosphine ligand(s) of the cobalt complex. Although the role of the excess phosphine is not known with certainty, the presence thereof in the reaction system appears to promote or otherwise modify catalyst activity. Phosphorus:cobalt atom ratios utilized in conjunction with the catalyst complex will range from 0.1:1 to 3:1, preferably from 0.5:1 to 2:1, more preferably from 1:1 to 1.5:1. A ratio of about 1.25:1 is particularly preferred.

In another modification of the process of the invention, an additional ruthenium catalyst is present in the catalyst system employed in the process of the invention. The ruthenium should be present in concentrations dependent upon that of the primary cobalt component. It should be present in Co:Ru atom ratio ranging from 1000:1 to 1:100, preferably from 100:1 to 1:10 and more preferably from 50:1 to 1:5.

The form of the ruthenium is not critical. Thus, it may be present in the form of a soluble homogeneous component or as a finely divided metal or supported on a carrier which is suspended in the reaction mixture or utilized in a fixed bed.

The soluble ruthenium components may be added in any of a number of forms including inorganic salts such as ruthenium nitrate, ruthenium sulfate, ruthenium fluoride, ruthenium chloride, ruthenium bromide, ruthenium iodide, ruthenium oxide and ruthenium phosphate or organic ruthenium salts such as ruthenium formate, ruthenium acetate, ruthenium propionate, ruthenium butyrate, ruthenium acetonylacetonate, etc., or aromatic ruthenium salts such as ruthenium benzoate, ruthenium phthalate, ruthenium naphthenate, etc., or as carbonyls such as bis-[ruthenium tricarbonyl dichloride] or bis-[ruthenium tricarbonyl dibromide], etc.

Ruthenium complexes are often more soluble than the salts and are, therefore, more desirable if high concentrations of homogeneous ruthenium solutions are desired. These complexes include ruthenium(III)tris-(2,4-pentanedionate), triruthenium dodecacarbonyl, ruthenium(II)dichlorotris-(triphenylphosphine), ruthenium(II)dichlorotetrakis-(triphenylphosphine), ruthenium(II)hydridochlorotris-(triphenylphosphine), or other soluble ruthenium complexes within the spirit of this group. Particularly suitable are ruthenium complexes of the phosphines described above which are used to form the cobalt carbonyl complexes.

The insoluble or heterogeneous ruthenium forms may be introduced as any of the forms given above which under a sufficiently hydrogen-rich atmosphere or reducing environment will give finely divided ruthenium. Alternatively, the insoluble ruthenium may be produced by reducing a soluble ruthenium form in the presence of a suitable support to give finely divided ruthenium deposited on supports including activated charcoal, alumina, silica gel, or zeolites. Other forms may be included if they can be divided finely enough by mechanical means such as ruthenium powder, ingot, shot, sponge, or wire.

The process of the invention is conducted in liquid-phase solution in an inert solvent. A variety of solvents which are inert to the reactants and catalyst and which are liquid at reaction temperature and pressure are in part operable. Illustrative of suitable solvent are hydrocarbons, particularly aromatic hydrocarbons of up to 16 carbon atoms such as benzene, toluene, xylene, ethylbenzene, and butylbenzene; alkanes such as hexanes, octanes, dodecanes, etc.; alkenes such as hexenes, octenes, dodecenes, etc.; alcohols such as t-butyl alcohol, hexanol, dodecanol, including alkoxylated alcohols; nitriles such acetonitrile, propionitrile, etc.; ketones, particularly wholly aliphatic ketones, i.e., alkanones, of up to 16 carbon atoms such as acetone, methyl ethyl ketone, diethyl ketone, methyl isobutyl ketone, ethyl hexyl ketone and dibutyl ketone; esters of up to 16 carbon atoms, particularly lower alkyl esters of carboxylic acids which are aliphatic or aromatic carboxylic acids having one or more carboxyl groups, preferably from 1 to 2, such as ethyl acetate, methyl propionate, propyl butyrate, methyl benzoate, diethyl glutarate, diethyl phthalate and dimethyl terephthalate; and ethers of up to 16 carbon atoms and up to 4 ether oxygen atoms, which ethers are cyclic or acyclic ethers and which are wholly aliphatic ethers, e.g., diethyl ether, diisopropyl ether, dibutyl ether, ethyl hexyl ether, methyl octyl ether, dimethoxyethane, diethylene glycol dimethyl ether, diethylene glycol diethyl ether, diethylene glycol dibutyl ether, tetraglyme, glycerol trimethyl ether, 1,2,6-trimethoxyhexane, tetrahydrofuran, 1,4-dioxane, 1,3-dioxane, 1,3-dioxolane and 2,4-dimethyl-1,3-dioxane, or which are at least partially aromatic, e.g., diphenyl ether, phenylmethyl ether, 1-methylnaphthalene, phenylisopropyl ether, halogenated hydrocarbons, such as chlorobenzene, dichlorobenzene, fluorobenzene, methyl chloride, methylene dichloride. Mixtures of solvents can also be utilized.

The amount of solvent to be employed is not critical. Typical molar ratios of reaction solvent to ethylene oxide reactant vary from 5:1 to 150:1.

Suitable selection of solvents can enhance product recovery. By selecting solvents with suitable polarity, a two phase system will form upon cooling of the reaction mixture with selective distribution of the catalyst and ligand in one phase and product 3-hydroxypropanal and 1,3-propanediol in a second phase. This will allow for easier separation of catalyst and ligand and recycle thereof back to the reactor. When a two phase separation process is used, solvents that would not be desirable in the reaction mixture, such as water and acids, can be used to enhance distribution of product to one phase and catalyst/ligand to the other phase.

Illustrative solvents for use in a one phase system are diethylene glycol, tetraglyme, tetrahydrofuran, t-butyl alcohol, and dodecanol. Illustrative solvents for use to provide a two phase system upon cooling are toluene, 1-methylnaphthalene, xylenes, diphenyl ether and chlorobenzene.

The process of the invention comprises contacting the 1,2-epoxide reactant and catalyst with carbon monoxide and molecular hydrogen. The molar ratio of carbon monoxide to hydrogen most suitably employed is from 4:1 to 1:6, with best results being obtained when ratios of from 1:1 to 1:4 are utilized. No special precautions need to be taken with regard to the carbon monoxide and hydrogen and commercial grades of these reactants are satisfactory. The carbon monoxide and hydrogen are suitable employed as separate materials although it is frequently advantageous to employ commercial mixtures of these materials, e.g., synthesis gas.

The addition of small amounts of acids and promoting metal salts to the hydroformylation reaction mixture can further enhance or promote the conversion of ethylene oxide by increasing the activity of the catalyst. Acids are defined herein to mean those compounds which can donate a proton under reaction conditions.

Suitable acids can include inorganic acids such HCl, HBr, HI, boric acid and organic acids in amounts ranging from trace amounts up to two times the molar amount of catalyst utilized. Suitable organic acids include the organo-acids having carbon numbers of 1 to 16, such as carboxylic acids, sulfonic acids, phosphonic acids, phosphinic acids as well as other organic compounds that will donate protons under reaction conditions such as imidazole, benzoimidazole, pyridinium salts, pyrazinium salts, pyrimidinium salts, particularly salts of the aforementioned acids. Non-limiting examples of organic acids include acetic acid, propionic acid, hexanoic acid, 2-ethylhexanoic acid, octanoic acid, 3-(phenylsulfonyl)-propionic acid, para-toluenesulfonic acid, 2-carboxyethylphosphonic acid, ethylphosphonic acid, n-butylphosphonic acid, t-butylphosphonic acid, phenylphosphonic acid, phenylphosphenic acid, phenyl boric acid, pyridinium para-toluenesulfonate and pyridinium octoate.

Another suitable method for providing promoter acids is to use as a catalyst precursor a cobalt salt of an organic acid, which will convert to cobalt carbonyl and the organic acid under reaction conditions. Such precursor salts include cobalt acetate, cobalt 2-ethylhexanoate, cobalt benzoate, cobalt formate and cobalt oleate. The ratio of gram equivalents of acid promoter to gram atoms of cobalt in the catalyst present in the reaction mixture will generally range from 0.001:1 to 4:1, preferably from 0.01:1 to 2:1.

Promoting amounts of metal salts can also be added to the reaction mixture along with the promoting amounts of acid to provide an even further enhanced promoting effect. Promoting amounts of one or more metal salts selected from a salt of a metal of Group IA, IIA, Group IIB, Group IIIB and the Rare Earth Series of the Periodic Table of the Elements (CAS version) are also added to the reaction mixture along with the promoting amounts of acid. Group IA comprises the alkali metals, lithium through cesium. Group IIA comprises the alkaline earth metals, calcium through barium. Group IIB comprises zinc, cadmium and mercury. Group IIIB comprises scandium, yttrium and lanthanum. The Rare Earth Group comprises cerium through lutetium. Any metal salt from the aforementioned Groups that is at least partially soluble in the reaction mixture is suitable. Both inorganic salts and organic salts are suitable. Included in the inorganic salts are halides, chromates, sulfates, borates, carbonates, bicarbonates, chlorates, phosphates, etc. Particularly desirable organic salts are salts of carboxylic acids having carbon numbers ranging from 1 to 20. Examples of metal salts that have been found suitable as copromoters include halides, such as bromides, iodides, and chlorides, carboxylates, such as acetates, propionates and octoates, borates, nitrates, and sulfates. In general a metal salt that does not react with the 1,2-epoxide, the reaction solvent or the hydroformylation products is suitable as copromoters with acids. The ratio of gram equivalents of metal of the salt promoter to gram atoms of cobalt in the catalyst present in the reaction mixture will generally range from 0.001:1 to 2:1, preferably from 0.01:1 to 1:1, and more preferably from 0.1:1 to 0.5:1.

In a preferred embodiment the product of the hydroformylation reaction is further hydrogenated to produce a product comprising substantially 1,3-diol. The hydroformylated product is preferably separated from the catalyst before being hydrogenated. Inert solvent may be added to the product prior to hydrogenation, or, if an inert (to hydrogenation) solvent was used in the hydroformylation reaction, it may be separated with the product and passed to the hydrogenation reactor. The hydrogenation catalyst can be any of the well known hydrogenation catalysts used in the art such as Raney nickel, palladium, platinum, ruthenium, rhodium, cobalt and the like. It is desirable to employ as the hydrogenation catalyst a metal or a compound of a metal which may be easily and economically prepared, which has a high degree of activity, and retains this activity for extended periods of time. The hydrogenation catalyst may be employed homogeneously, in a finely divided form and dispersed throughout the reaction mixture, or preferably it may be employed on a support or carrier material such as alumina and carbon. Preferred catalysts are Raney nickel and supported platinum, particularly platinum on carbon. Hydrogenation conditions include pressures ranging from 345 to 68948 kPa (50 to 10,000 psi) and temperatures ranging from 30 to 175°C. The hydrogenating gas used is molecular hydrogen or a mixture of hydrogen and carbon monoxide such as that used for the hydroformylation reaction.

The ranges and limitations provided in the instant specification and claims are those which are believed to particularly point out and distinctly claim the instant invention.

### Illustrative Embodiment I

In the examples and tables the following abbreviations are used:
- EO/PDO/3-HPA: ethylene oxide/1,3-propanediol/3-hydroxypropanal,
- PO/BDO/3-HBA: propylene oxide/1,3-butanediol/3-hydroxybutanal,
- 9-PHOSPHA: 1,2-bis(9-phosphabicylcononyl)ethane,
- DIPHOS: 1,2-bis(diphenylphosphino)ethane,
- BDCHP: 1,2-bis(dicyclohexylphosphino)ethane,
- TBP: tri-n-butylphosphine, and
- TPP: triphenylphosphine.
"EO Conv. Rate" refers to the rate in grams of EO converted per hour."PDO Precursors" are those compounds which upon hydrogenation produce PDO and include primarily 3-HPA with smaller amounts of dimers, trimers and other oligomers of 3-HPA being present. Also included are small amounts of acrolein and propionaldehyde. Most of the acrolein is an artifact of the gas chromatographic (GC) measuring process as a result of decomposition of 3-HPA during analysis. In situ infrared spectroscopic analyses after completion of the hydroformylation reactions showed no acrolein present in the products. Lowering the temperature and chemically passifying the injection port of the GC instrument dramatically lowered these artifact peak heights, indicating that it is at most only a minor product (1-2%).

In this illustrative embodiment catalysts complexed with the preferred ligands are prepared and tested for hydroformylation of ethylene oxide and compared with catalysts prepared from non-preferred ligands.

### In Situ Catalyst Preparation and Hydroformylation:

### Examples 1-2

In an inert atmosphere, a 100 ml air-stirred Parr autoclave was charged with 228 mg (0.66 mmole) of cobalt octoate, 155 mg (0.50 mmole) of 9-PHOSPHA (as a mixture of [4.2.1] and [3.3.1] isomers) and 23 ml of dry, nitrogen-purged toluene-clorobenzene solution (5:1 volume ratio). The autoclave was sealed and pressured to 9065 kPa (1300 psig) with a hydrogen-carbon monoxide gas mixture (1:1 molar ratio). The reaction was stirred and heated at 130°C for 30 minutes at 10443 kPa (1500 psig). The reactor was then cooled to an internal temperature of 5°C and the gases were vented to leave the autoclave at ambient pressure.

EO (4.5 g, 102 mmole) was added to the reactor and the reactor was then heated and stirred for 3 hours at 105°C at a pressure of 9754 to 10443 kPa (1400 to 1500 psig) of hydrogen-carbon monoxide gas (1:1 molar ratio).

After cooling to 5°C, the reactor was purged with nitrogen and the two-phase product mixture was collected to give about 29 grams of solvent phase and about 2 grams of an oil phase. The two phases were independently analyzed by gas chromatography. Results are shown in Table 1 as Example 1.

Example 1 was repeated except that the hydrogen/carbon monoxide ratio was changed to 4:1. The results are presented in Table 1 as Example 2.

**TABLE 1**

| | | | | --- mole % selectivity --- | | |
|---|---|---|---|---|---|---|
| Example | H₂/CO Ratio | EO Conv. Rate | EO Conv. Mole % | PDO Precursor | PDO | CH₃CHO |
| 1 | 1:1 | 0.37 | 21.2 | 93.4 | 0.8 | 5.8 |
| 2 | 4:1 | 0.48 | 31.2 | 92.3 | 2.1 | 5.6 |

### Examples 3-6

The hydroformylation of ethylene oxide was repeated using the procedure, catalyst and conditions described in Example 1 except that varying amounts of the 9-PHOSPHA ligand to cobalt molar ratios were used. The results are shown in Table 2.

**TABLE 2**

| | | | | --- mole % selectivity --- | | |
|---|---|---|---|---|---|---|
| Example | P/Co Ratio | EO Conv. Rate | EO Conv. mole % | PDO Precursor | PDO | CH₃CHO |
| 3 | 2.0 | 0.21 | 13.9 | 75.4 | 9.9 | 6.9 |
| 4 | 1.5 | 0.45 | 25.1 | 93.1 | 1.3 | 6.0 |
| 5 | 1.25 | 0.46 | 32.1 | 88.9 | 1.5 | 8.9 |
| 6 | 1.0 | 0.74 | 45.0 | 76.8 | 2.1 | 18.6 |

### Examples 7-11

The hydroformylation of EO was repeated using the procedure described in Example 1 with the following differences: reaction temperature and ligands were changed in different examples and indicated in Table 3. Results are shown in Table 3. Phosphine ligands, not of this inventions, were also tested and the results are shown in Table 3 (C-1 to C-8).

**TABLE 3**

| | | | | | -- mole % selectivity -- | | |
|---|---|---|---|---|---|---|---|
| Examples | Ligand ^{a)} | Rxn Temp. °C | EO Conv. Rate | EO Conv. Mole % | PDO | PDO Precursor | CH₃CHO |
| C-1 | TBP | 115 | | 3.2 | - | 21.0 | 69.0 |
| C-2 | TBP | 105 | | 1.8 | - | 30.0 | 70.0 |
| C-3 | DIPHOS | 105 | 0.74 | 54.0 | 3.8 | 80.8 | 13.7 |
| 7 | 9-PHOSPHA | 105 | 0.45 | 25.1 | 1.3 | 91.9 | 6.0 |
| C-4 | # | 105 | 0.89 | 47.6 | 3.2 | 78.3 | 18.1 |
| C-5 | DIPHOS | 100 | 0.64 | 44.6 | 2.3 | 88.1 | 8.4 |
| 8 | 9-PHOSPHA | 100 | 0.37 | 21.3 | 0.9 | 95.8 | 4.5 |
| C-6 | DIPHOS | 95 | 0.43 | 31.2 | 1.6 | 91.3 | 5.8 |
| 9 | 9-PHOSPHA | 95 | 0.27 | 15.4 | 0.8 | 96.5 | 3.7 |
| C-7 | DIPHOS | 90 | 0.32 | 22.0 | 3.1 | 89.0 | 6.4 |
| 10 | 9-PHOSPHA | 90 | 0.20 | 11.5 | 0.6 | 98.5 | - |
| C-8 | = | 90 | 0.27 | 19.0 | 0.3 | 94.9 | - |
| 11 | @ | 90 | 0.29 | 14.1 | 9.0 | 81.6 | 9.3 |

| | | | | | | | |
|---|---|---|---|---|---|---|---|
| a) # = 1-(9-phosphabicyclononyl)-2-(diphenylphosphino)ethane. @ - 1,3-bis(9-phosphabicyclononyl)propane. | | | | | | | |

### Examples 12-15

The hydroformylation of ethylene oxide was repeated using the procedure of Example 1 except that dicobalt octacarbonyl (0.33 mmoles) was used as the cobalt source and various molar ratios of 9-PHOSPHA to cobalt was used. Results are shown in Table 4.

**TABLE 4**

| | | | | --- mole % selectivity --- | | |
|---|---|---|---|---|---|---|
| Example | P/Co Ratio | EO Conv. Rate | EO Conv. Mole % | PDO Precursor | PDO | CH₃CHO |
| 12 | 2.0 | 0.01 | 0.7 | 100 | - | - |
| 13 | 1.5 | 0.03 | 1.8 | 100 | - | - |
| 14 | 1.0 | 0.17 | 8.9 | 70.3 | 1.3 | 0.2 |
| 15 | 1.0 | 0.27 | 15.8 | 74.3 | 1.8 | - |

### Example 16-18

The hydroformylation of EO was repeated using the procedure (and ligand) of Example 1 except that cobalt octacarbonyl was used as the cobalt source. Various acids were used as promoters, except for the control experiment in which no acid was used. The results are shown in Table 5.

**TABLE 5**

| | | | | --- mole % selectivity --- | | |
|---|---|---|---|---|---|---|
| Example | Acid, mmoles | EO Conv. Rate | EO Conv. Mole % | PDO Precursor | PDO | CH₃CHO |
| 16 | A,0.165 | 0.20 | 11.3 | 93.7 | - | - |
| 17 | B,0.165 | 0.19 | 11.4 | 91.9 | 1.2 | 4.5 |
| 18 | B,0.33 | 0.25 | 13.6 | 92.0 | - | 4.8 |
| Control | - | 0.04 | 2.1 | 100.0 | - | - |
| A = pyridinium para-toluene sulfonate | | | | | | |
| B = n-octanoic acid | | | | | | |

The following acids when used as a promoter in the above reaction were also found to increase the amount of EO converted (mole %): 2-carboxyethylphosphonic acid (13%), n-butylphosphonic acid (16%), phenyl- phosphonic acid (14.5%), t-butylphosphonic acid (18.5%), 3-(phenylsulfonyl)-propionic acid (22%), phenylboric acid (18%), phenylphosphenic acid (36%), and imidazole (26%).

### Examples 19 and 20

The hydroformylation of EO was repeated using the procedure (and ligand) of Example 1 except a reaction temperature of 90°C was used. In Example 19 cobalt 2-ethylhexanoate was used as a cobalt source and 0.21 mmoles of sodium acetate was added in the catalyst preparation step as a catalyst copromoter. In Example 20 dicobalt octacarbonyl was used as a cobalt source and 1.32 mmoles of 2-ethylhexanoic acid and 0.21 mmoles of sodium acetate were added as catalyst promoters. The results are shown in Table 6.

**TABLE 6**

| | | | - mole % selectivity - | |
|---|---|---|---|---|
| Example | EO Conv. Rate | EO Conv. Moles % | PDO Precursor | CH₃CHO |
| 19 | 0.65 | 39.2 | 96.5 | 3.1 |
| 20 | 0.64 | 40.1 | 98.6 | 3.1 |

### Illustrative Embodiment II: Hydrogenation of Hydroformylation Product:

### Example 21

Ten grams of the reaction product from an EO hydroformylation reaction such as example 1 above was charged to a 300 ml autoclave along with 40 grams of deionized water and 2 grams of Raney nickel catalyst. The autoclave was flushed with hydrogen, pressured with hydrogen to 6996 kPa (1000 psig) and heated to 110°C for 5 hours. The autoclave was cooled to room temperature, vented of excess gas, and samples were removed for analysis by gas chromatography and mass spectroscopy. This analysis showed PDO as the major product in the water solution. The selectivity to PDO was estimated to be about 90% with greater than 80% of 3-HPA converted to the diol.

### Example 22

Example 21 was repeated except 18 grams of hydroformylation product, 42 grams of water and 1.5 grams of molybdenum-promoted Raney nickel catalyst were charged to the autoclave with a reaction pressure of 4137 kPa (600 psi) and a reaction temperature of 60°C being used. Analysis of the hydrogenated product showed that 3-HPA was converted to PDO as the major product.

### Illustrative Embodiment III

In this illustrative embodiment catalysts complexed with the preferred ligands promoted by the acid/salt promoter of the instant invention are prepared and tested for hydroformylation of EO and compared with catalysts which are not promoted by the promoter system of the instant invention.

### In situ catalyst preparation and hydroformylation:

### Example 23, 24

Example 1 was repeated, except that 113 mg (0.33 mmole) of dicobalt octacarbonyl and 117 mg (0.21 mmole) of sodium acetate were used. The result is shown in Table 7.

Example 23 was repeated except that 228 mg (0.66 mmole) of cobalt 2-ethylhexanoate was used as the cobalt source. The results (example 24) are presented in Table 7. The use of cobalt acetate as the cobalt provides similar results.

### Examples 25 to 34, C-9 to C-13

Examples 25-34 in Table 7 are examples carried out in the same fashion as example 23, but using 0.66 mmole of cobalt 2-ethylhexanoate as cobalt source and different salts as the salt promoter.

Examples C-9 through C-13 in Table 7 are comparative examples in respect of embodiment III, carried out in the same fashion as example 23, but without the acid promoter, the salt promoter or both the acid and salt promoters.

### Examples 35 to 41

Example 24 was repeated but using different amounts of salt promoter. These results are shown in Table 8.

### Examples 42 to 43

Example 24 was repeated at the temperatures indicated in Table 9 and at hydrogen to carbon monoxide ratio of 4:1 and a phosphorous to cobalt ratio in the catalyst of 1.25:1. The results are shown in Table 9.

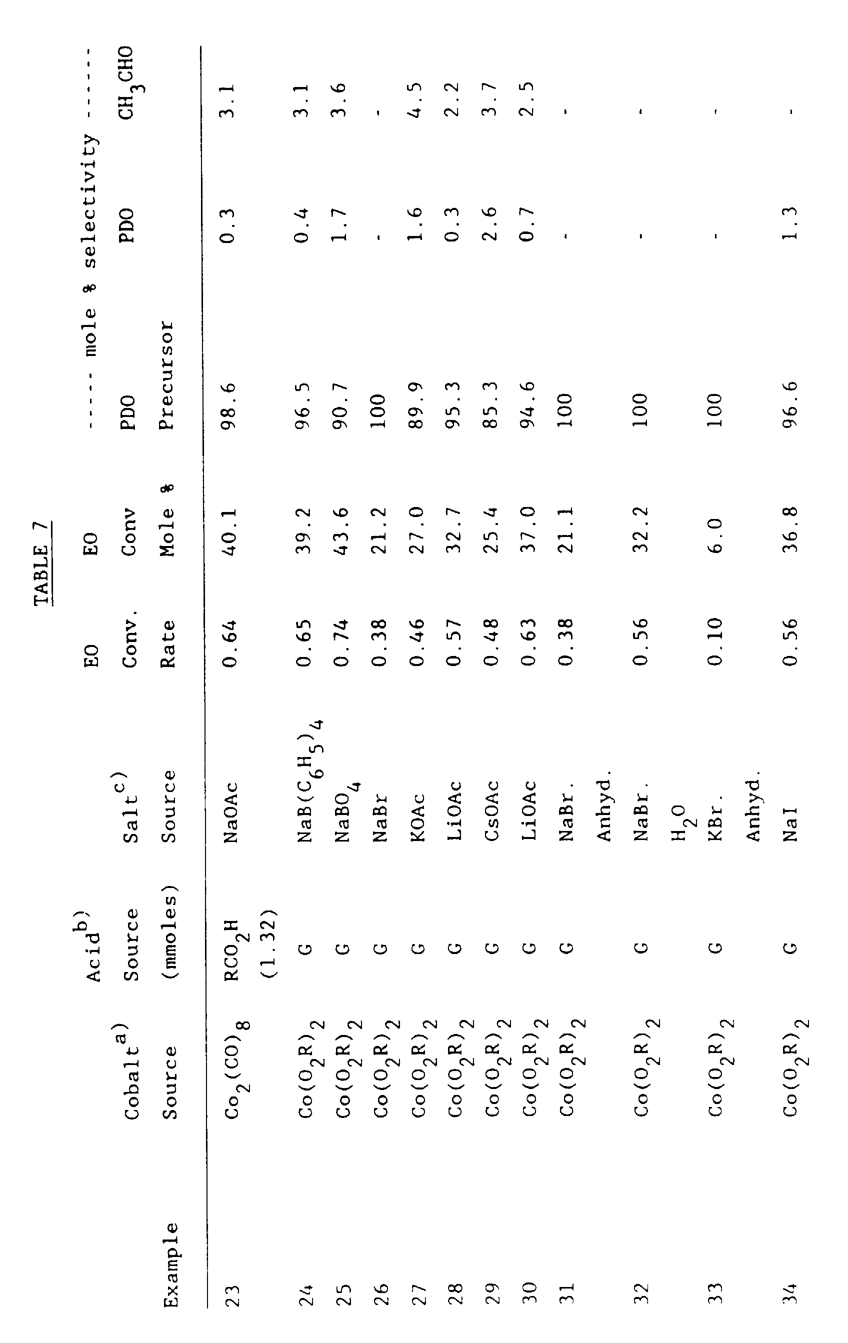

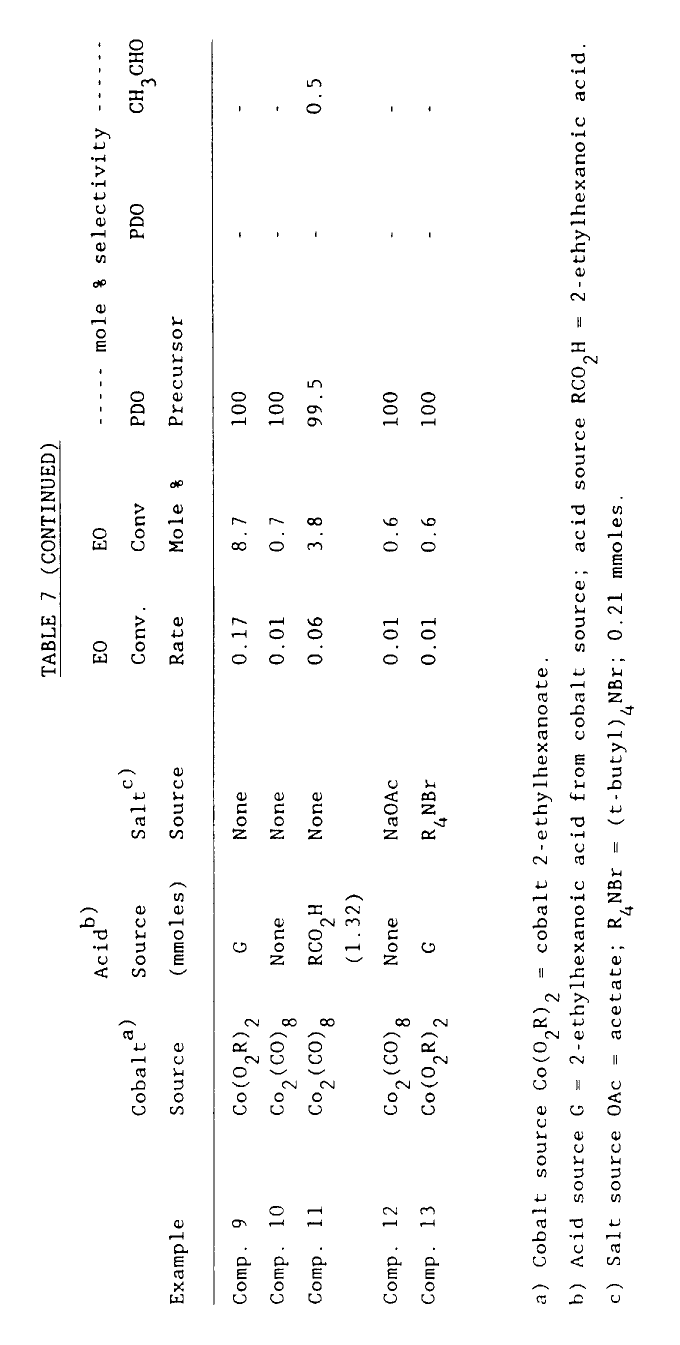

**TABLE 8**

| | | | | --- mole % selectivity --- | | |
|---|---|---|---|---|---|---|
| Example | NaOAc^{a)} (mmoles) | EO Conv. Rate | EO Conv. Mole % | PDO Precursor | PDO | CH₃CHO |
| 35 | 1.0 | 0.61 | 36.4 | 62.5 | 0.2 | 4.8 |
| 36 | 0.66 | 0.70 | 39.6 | 60.4 | 2.5 | 4.1 |
| 37 | 0.42 | 0.68 | 41.7 | 66.6 | 2.0 | 4.4 |
| 38 | 0.21 | 0.68 | 39.3 | 94.3 | 0.8 | 2.5 |
| 39 | 0.10 | 0.56 | 30.2 | 97.5 | 0.2 | 2.3 |
| 40 | 0.05 | 0.30 | 17.2 | 96.1 | 0.5 | 2.4 |
| 41 | None | 0.17 | 8.7 | 100 | - | - |

| | | | | | | |
|---|---|---|---|---|---|---|
| a) NaOAc = Sodium acetate. | | | | | | |

**TABLE 9**

| | | | | --- mole % selectivity --- | | |
|---|---|---|---|---|---|---|
| Example | Rxn Temp. °C | EO Conv. Rate | EO Conv. Mole % | PDO Precursor | PDO | CH₃CHO |
| 42 | 80 | 0.77 | 43.4 | 97.3 | 2.7 | - |
| 43 | 90 | 0.48 | 27.3 | 97.8 | 2.2 | - |

### Examples 44 to 46

Example 24 was repeated at 80°C and at the pressure indicated in Table 10. The results are shown in Table 10.

**TABLE 10**

| | | | | --- mole % selectivity --- | | |
|---|---|---|---|---|---|---|
| Example | Pressure kPa | EO Conv. Rate | EO Conv. Mole % | PDO Precursor | PDO | CH₃CHO |
| 44 | 10342 | 0.77 | 43.4 | 97.4 | 2.7 | - |
| 45 | 6895 | 0.94 | 50.4 | 94.3 | 3.4 | 2.3 |
| 46 | 4137 | 0.59 | 34.1 | 75.5 | 3.8 | 4.7 |

### Illustrative Embodiment IV

In this illustrative embodiment the oxidation of the complexing phosphine ligand is illustrated.

1.56 Grams of 9-PHOSPHA was loaded into a 100 ml three neck round bottom flask, fitted with a rubber septum, thermometer and gas stopcock. A stir bar was added, and 25 ml. of ethanol was poured into the flask. The mixture was then degassed with argon. An argon filled balloon was attached to the gas inlet and the mixture was stirred for 15 minutes at room temperature. 0.36 Grams of (30% by vol.) aqueous hydrogen peroxide solution was weighed into a 10 ml two neck flask fitted with a rubber septum and gas stopcock. Five ml. of ethanol was added via a syringe through the septum and the mixture was then degassed with argon. A length of teflon tubing for a cannula was cut and each end was threaded through a 12 gauge needle. The rubber septum on the smaller flask was pierced with one needle and the needle was then pulled out, leaving the tubing in place above the liquid level. The argon flow was turned on to flush through the tubing. The septum on the larger flask was pierced with the other needle, then the needle was withdrawn, leaving the tubing in place approximately 0.5 inches above the liquid level. The tubing in the small flask was carefully inserted into the liquid as far as possible and using the argon flow, the flow of liquid from the peroxide mixture into the ligand mixture was regulated so that it was transferred drop by drop. The solution was stirred at room temperature for one hour.

The solution was then transferred to a 250 ml. round bottom flask in a nitrogen atmosphere, using degassed ethanol to rinse out the solids. The ethanol was removed from the solution using a rotovapor, then the solid was dried under vacuum for several hours. The resulting oxidized phosphine ligand was analyzed by phosphorus-31 NMR to determine the oxygen:phosphorus ratio.

### In situ catalyst preparation and hydroformylation:

### Examples 47, 48-59

Example 1 was repeated, except that 113 mg (0.33 mmole) of dicobalt octacarbonyl and 204 mg (0.66 mmole) of 9-PHOSPHA which had been oxidized as described above to provide an oxygen to phosphorus of 0.20 were used. The result is shown in Table 11.

Example 47 was repeated except that differing amounts of ligand to dicobalt octacarbonyl, differing reaction temperatures and differing amounts of oxidation of the phosphine ligand were used. The results are presented in Table 11 as Examples 48-59.

**TABLE 11**

| | | | | | --- mole % selectivity --- | | |
|---|---|---|---|---|---|---|---|
| Example | O/P Ratio | P/Co Ratio | Rxn Temp. °C | EO Conv. Mole % | PDO Precursor | PDO | CH₃CHO |
| 47 | 0 | 2.0 | 105 | 1.0 | 100 | - | - |
| 48 | 0 | 1.5 | 105 | 3.7 | 89.0 | - | 11.0 |
| 49 | 0 | 2.0 | 105 | 0.7 | 100 | - | - |
| | Trace | | | | | | |
| 50 | P = 0 | 2.0 | 105 | 3.4 | 100 | - | - |
| 51 | 0.07 | 1.9 | 105 | 8.7 | 94.7 | - | 5.3 |
| 52 | 0.07 | 2.0 | 105 | 10.1 | 98.5 | 1.5 | - |
| 53 | 0.13 | 2.0 | 110 | 17.7 | 86.3 | 1.9 | 4.9 |
| 54 | 0.20 | 2.5 | 105 | 21.2 | 90.1 | 3.6 | 4.0 |
| 55 | 0.20 | 2.0 | 105 | 33.9 | 86.9 | 3.2 | 7.2 |
| 56 | 0.20 | 1.5 | 105 | 42.9 | 65.9 | 1.5 | 18.2 |
| 57 | 0.20 | 2.0 | 100 | 20.9 | 90.3 | 4.0 | 4.0 |
| 58 | 0.30 | 1.4 | 105 | 47.0 | 81.3 | 3.3 | 15.5 |
| 59 | 0.30 | 1.4 | 90 | 30.3 | 90.0 | 4.2 | 3.8 |

### Illustrative Embodiment V

### In situ catalyst preparation and hydroformylation:

### Example 60

Example 1 was repeated, however, using 228 mg (0.66 mmole) of cobalt 2-ethylhexanoate, 221 mg (0.66 mmole) of 9-PHOSPHA and 74 mg of [Ru(CO)₃Cl₂]₂ (0.29 mmoles of ruthenium, basis metal). The result is shown in Table 12 as Example 60.

### Comparative Example C-14

The above example was repeated, however, without using the ruthenium co-catalyst. The results are shown in Table 12 as example C-14. (comparative example in respect of embodiment V)

### Examples 61-67

Example 65 was repeated varying the promoter salt used, the phosphine ligand used and the ruthenium compound used. These variations and the results are shown in Table 12.

### Examples 68-74

Example 60 was repeated using different promoter metal salts and a reaction time of 1.5 hours. Triruthenium dodecacarbonyl was used as the source of ruthenium. The results are shown in Table 13.

### Example 75

Example 60 was repeated except a 130 mg (0.42 mmoles) of 9-PHOSPHA, 17 mg (0.21 mmoles) of sodium acetate and 1.0 g of finely divided activated carbon having deposited on its surface 5 %wt of ruthenium metal were used. The EO converted was 63.2 %mole and the selectivity to 3-HPA was 5.0 %mole and to PDO was 54.9 %mole.

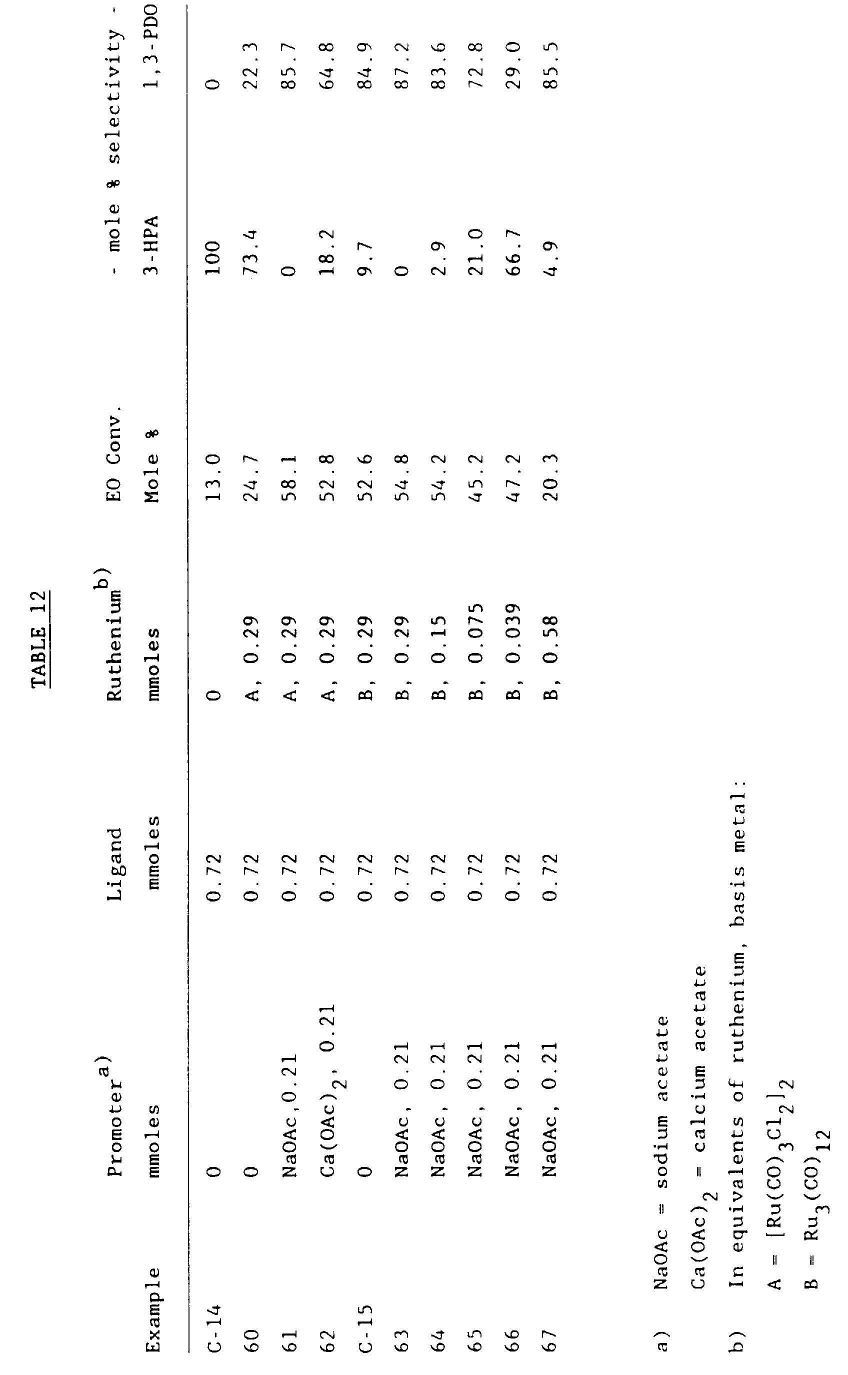

**TABLE 13**

| | | | - mole % selectivity - | |
|---|---|---|---|---|
| Example | Promoter | EO Conv. Mole % | 3-HPA | 1,2-PDO |
| 68 | Yb(OAc)₃ | 59.5 | 17.5 | 76.3 |
| 69 | Eu(OAc)₃ | 50.8 | 17.1 | 75.0 |
| 70 | Y(OAC)₃ | 62.2 | 19.9 | 74.5 |
| 71 | Er(OAc)₃ | 53.2 | 19.3 | 73.3 |
| 72 | La(OAc)₃ | 39.5 | 17.6 | 74.9 |
| 73 | Zn(Oac)₂ | 33.0 | 4.0 | 89.2 |
| 74 | Ca(OAc)₂ | 30.7 | 17.8 | 74.2 |

### Illustrative Embodiment VI

In this illustrative embodiment catalysts complexed with the preferred ligands promoted by the acid/salt promoter of the instant invention are prepared and tested for hydroformylation of EO and compared with catalysts which are not promoted by the promoter system of the instant invention.

### In Situ Catalyst Preparation and Hydroformylation:

Example 1 was repeated, however, using 228 mg (0.66 mmole) of cobalt 2-ethylhexanoate, 155 mg (0.50 mmole) of 9-PHOSPHA and 33 mg (0.21 mmole) of calcium acetate. The result is shown in Table 14 as example 80.

The above example was repeated using differing amounts of promoter salt and different promoter salts with the difference that a reaction time of 1.5 rather than 3 hours was used for examples 82 through 87. The results are shown in Table 14.

Example C-16 in Table 14 is a comparative example in respect of embodiment VI, carried out in the same fashion as above, but without the salt promoter.

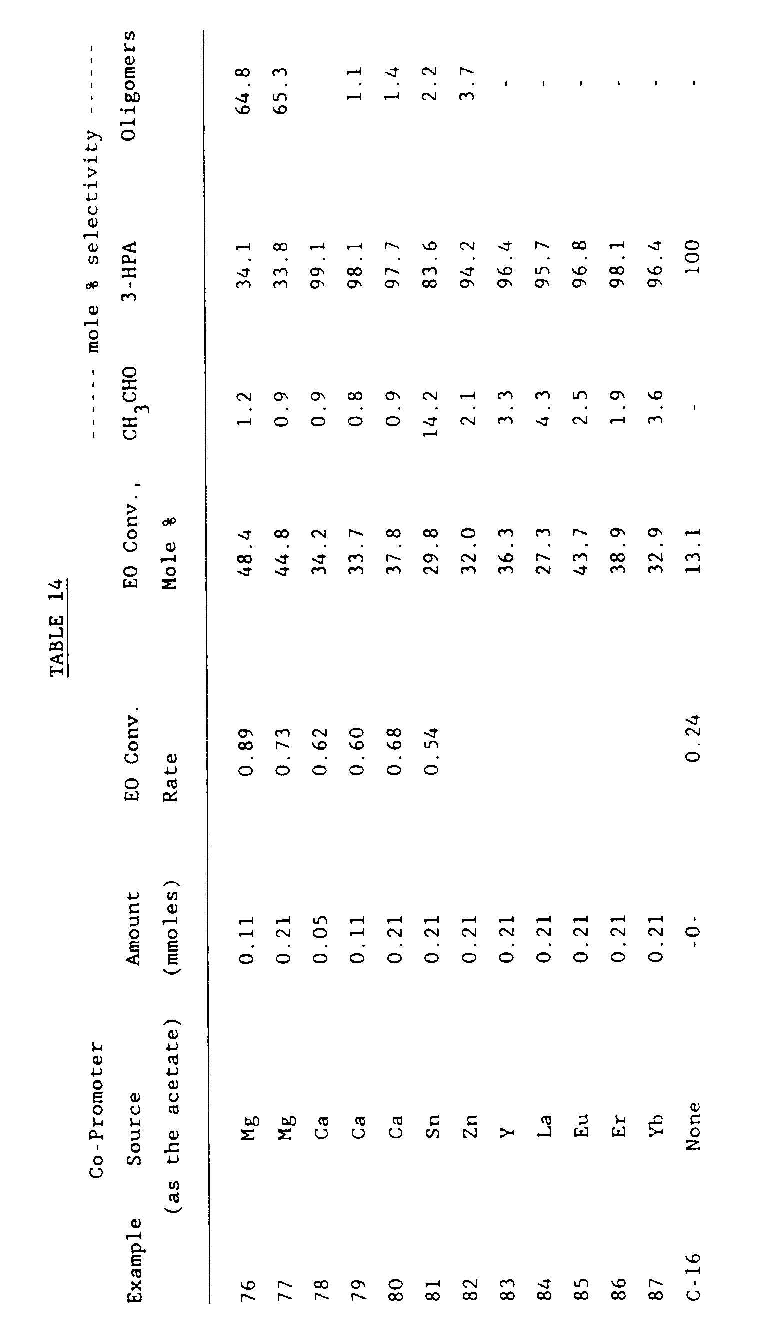

### Illustrative Embodiment VII

In this illustrative embodiment catalysts complexed with the preferred ligands are prepared and tested for hydroformylation of PO and compared with catalysts prepared from non-preferred ligands.

### In Situ Catalyst Preparation and Hydroformylation of PO:

### Example 88

Example 1 was repeated, however, using 6.6 g (0.11 mole) of PO instead of EO and heating the reaction mixture to 90°C for 3 hours. The homogeneous reaction mixture was removed from the autoclave to give 29.7 g of a clear liquid. The reaction mixture was analyzed by gas chromatography and GC/mass spectrometry which showed 4.7% conversion of PO to 81.0% 3-HBA and a small amount of 2-butenal by-product. The 2-butenal is thought to be an artifact of the GC analysis, resulting from decomposition of the 3-HBA on the GC column. The small amounts of 2-butenal observed upon analysis has been added to the 3-hydroxybutanal selectivities listed in the Table 15.

### Examples 89-97, C17-C18

Additional examples of PO hydroformylation were carried out using different cobalt precursors, different salt and acid catalyst promoters and different temperatures and these are shown in Table 15. Examples C17-18 are comparative in respect of Embodiment VII.

### Examples 98 to 106

Example 1 was repeated, however, using 62 mg (0.097 mmol) of trirutheniumdodecacarbonyl, 18 mg (0.21 mmol) of sodium acetate, 207 mg (0.66 mmole) of 9-PHOSPHA and 6.6 g (0.11 mole) of PO. The reaction mixture was stirred and heated at 90°C for 3 hours. The reaction mixture was removed from the autoclave to give 25.6 g of a clear, low viscosity upper phase liquid and 3.1 g of a clear, more viscous lower phase liquid. The two phases were analyzed by gas chromatography and GC/mass spectrometry which showed 19% conversion of PO to products. The reaction product distribution was 2% acetone (rearrangement product of PO), 9% 3-HBA, 87% 1,3-butanediol and 2% of miscellaneous products of which the majority was propene. These results are summarized in Table 16 as example 99. A series of other experiments, including comparative experiments. were run under similar conditions and are shown in Table 16.

### Illustrative Embodiment VIII

### In Situ Catalyst Preparation and Hydroformylation of 1.2-Epoxyhex-5-ene and 1,2-Epoxyhexane

### Example 107

Example 1 was repeated, however, using 11.4 g (0.11 mole) of 1,2-epoxyhex-5-ene instead of EO and heating the reactor to 90°C for 3 hours. The reaction mixture was removed from the autoclave to give 38.6 g of a homogeneous, clear, amber liquid. The reaction mixture was analyzed by gas chromatography and GC/Mass Spectrometry which showed 9.2% conversion of 1,2-epoxyhex-5-ene to 81.0% 3-hydroxyhept-6-enal and several minor unidentified by-products.

### Examples 108 and 109

Example 1 was repeated, however, using 62 mg (0.097 mmole) of trirutheniumdodecacarbonyl, 18 mg (0.21 mmole) of sodium acetate, 207 mg (0.66 mmole) of 9-PHOSPHA and 11.4 g (0.11 mole) of 1,2-epoxyhexane. The reaction was stirred and heated at 90°C for 3 hours. The reaction mixture was removed from the autoclave to give 33.15 g of a homogeneous, clear liquid. The reaction mixture was analyzed by gas chromatography and GC/Mass Spectrometry which showed 8% conversion of 1,2-epoxyhexane to products. The reaction product distribution was 5% 2-hexanone (rearrangement product of 1,2-epoxyhexane), 50% 3-hydroxyheptanal, 18% 1,3-heptanediol and 27% of miscellaneous products consisting of 1-hexene, pentanal and other unidentified products.

Another experiment was run under the same conditions with the exception that the sodium acetate was deleted. Analysis showed 5% conversion of 1,2-epoxyhexane to products. The reaction product distribution was 3% 2-hexanone (rearrangement product of 1,2-epoxyhexane), 78% 3-hydroxyheptanal, 0% 1,3-heptanediol and 19% of miscellaneous products consisting of 1-hexene, pentanal and other unidentified products.

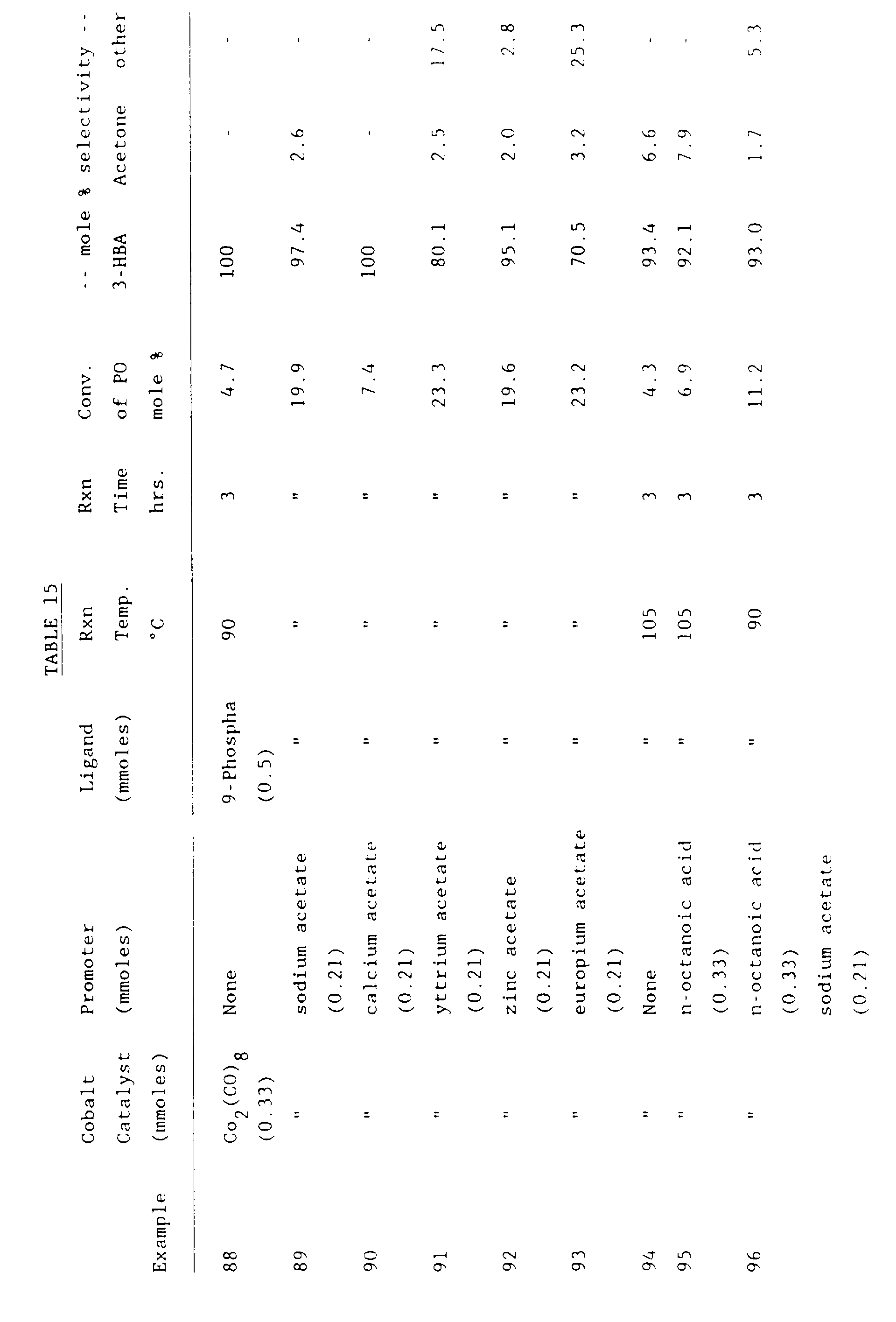

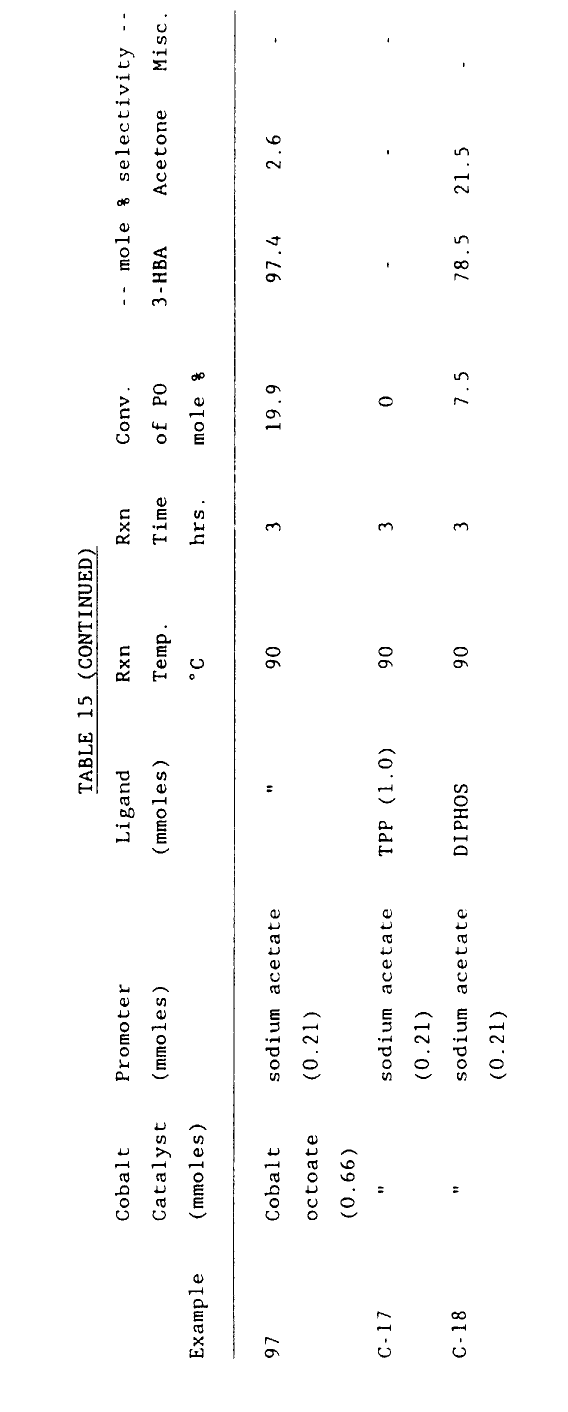

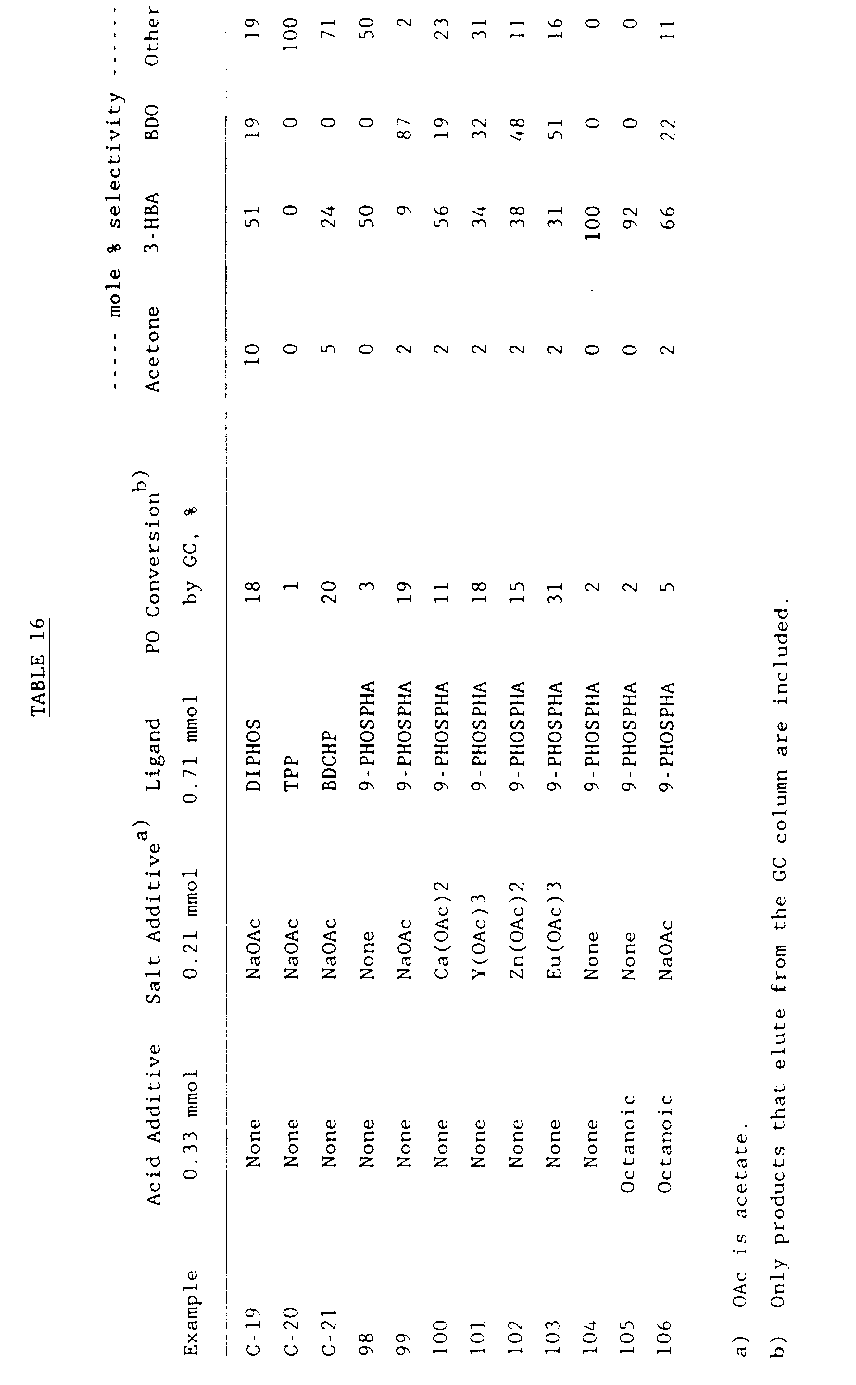

## Claims

1. A process for making 1,3-diols and 3-hydroxyaldehydes by hydroformylating 1,2-epoxides which process comprises intimately contacting
(a) a 1,2-epoxide having at least 2 carbon atoms,
(b) ditertiary phosphine-modified cobalt carbonyl catalyst, said phosphine comprising a hydrocarbylene-bis(monophosphabicycloalkane) in which each phosphorus atom is joined to hydrocarbylene and is a member of a bridge linkage without being a bridgehead atom and which hydrocarbylene-bis(monophosphabicycloalkane) has 11 to 300 carbon atoms; of which 5 to 12 carbon atoms together with a phosphorus atom are members of each of the two bicyclic skeletal structures,
(c) carbon monoxide, and
(d) hydrogen, the molar ratio of carbon monoxide to hydrogen being
from 4:1 to 1:6, preferably from 1:1 to 1:4 in liquid-phase solution in an inert reaction solvent, at a temperature of from 30 to 150°C and a pressure of from 345 to 68948 kPa (50 to 10,000 psi).

2. The process of claim 1, wherein the catalyst comprises a catalyst promotor.

3. The process of claim 2, wherein the catalyst promotor comprises an acid.

4. The process of claim 3, wherein the ratio of gram equivalents of acid to gram atoms of cobalt in the catalyst ranges from 0.001 to 4:1, preferably from 0.01 to 2:1.

5. The process of any one of claims 2 to 4, wherein the catalyst promotor comprises a metal salt promotor selected from a salt of a metal of Group IA, Group IIA, Group IIB, Group IIIB and the Rare Earth Series of the Periodic Table of the Elements (CAS version).

6. The process of claim 5, wherein the ratio of gram equivalents of metal salt to gram atoms of cobalt in the catalyst ranges from 0.001:1 to 2:1, preferably from 0.01 to 1:1, more preferably, from 0.1:1 to 0.5:1.

7. The process of any one of claims 1 to 4, wherein an additional ruthenium catalyst is present.

8. The process of claim 7, wherein the Co:Ru atom ratio ranges from 1000:1 to 1:100, preferably from 100:1 to 1:10, more preferably from 50:1 to 1:5.

9. The process of any one of claims 1 to 8, wherein the phosphine, prior to being complexed with said cobalt carbonyl catalyst, is partially oxidized to provide an oxygen to phosphorus ratio ranging from 0.01:1 to 0.5:1.

10. The process of claim 9, wherein the phosphine is partially oxidized to provide an oxygen to phosphorus ratio ranging from 0.05:1 to 0.3:1

11. The process of any one of claims 1 to 10, wherein the 1,2-epoxide is selected from 1,2-epoxyalkanes or 1,2-epoxyalkenes having carbon numbers ranging from 2 to 30, preferably from 2 to 20, more preferably from 2 to 10.

12. The process of claim 11, wherein the 1,2-epoxide is ethylene oxide or propylene oxide, preferably ethylene oxide.

13. The process of any one of claims 1 to 12, wherein the phosphine is a α,Ω-hydrocarbylene-P,P'-bis(monophosphabicyclononane) in which ring systems (a) each phosphorus atom is a member of a bridge linkage, (b) each phosphorus atom is not in a bridgehead position, and (c) each phosphorus atom is not a member of the bicyclic system of the other, and (d) the smallest phosphorus-containing rings contain at least four, preferably at least five atoms.

14. The process of any one of claims 1 to 13, wherein the phosphine is of the formula 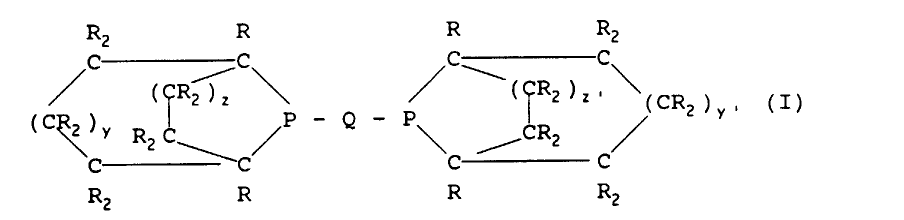 where Q represents hydrocarbylene of up to 30 carbon atoms; y and z represent zero or positive integers whose sum is from 0 to 7; y' and z', independent of the values of y and z, represent zero or positive integers whose sum is from 0 to 7; and R independently represents hydrogen and alkyl of from 1 to 30 carbon atoms.

15. The process of any one of claims 1 to 14, wherein in the phosphine Q is selected from ethylene, propylene and butylene.

16. The process of any one of claims 1 to 15, wherein in the phosphine the monophosphabicyclononane is selected from 9-phosphabicyclo[4.2.1]nonane, 9-phosphabicyclo[3.3.1]nonane and mixtures thereof.

17. The process of any one of claims 1 to 16, wherein the phosphorous to cobalt atom ratio in the catalyst ranges from 0.1:1 to 3:1, preferably 0.5:1 to 2:1, more preferably from 1:1 to 1.5:1.

## Patentansprüche

1. Verfahren zur Herstellung von 1,3-Diolen und 3-Hydroxyaldehyden durch Hydroformylieren von 1,2-Epoxiden, welches Verfahren ein inniges Inkontaktbringen von
(a) einem 1,2-Epoxid mit wenigstens 2 Kohlenstoffatomen,
(b) einem ditert.-Phosphin-modifizierten Kobaltcarbonylkatalysator, welches Phosphin ein Hydrocarbylen-bis(monophosphabicycloalkan) umfaßt, worin jedes Phosphoratom an Hydrocarbylen gebunden ist und ein Glied einer Brückenverknüpfung darstellt, ohne ein Brückenkopfatom zu sein, und welches Hydrocarbylenbis(monophosphabicycloalkan) 11 bis 300 Kohlenstoffatome enthält, von denen 5 bis 12 Kohlenstoffatome zusammen mit einem Phosphoratom Glieder jeder der beiden bicyclischen Skelettstrukturen sind,
(c) Kohlenmonoxid und
(d) Wasserstoff, wobei das Molverhältnis von Kohlenmonoxid zu wasserstoff von 4:1 bis 1:6, vorzugsweise von 1:1 bis 1:4 beträgt,
in Flüssigphasenlösung in einem inerten Reaktionslösungmittel bei einer Temperatur von 30 bis 150°C und einem Druck von 345 bis 68.948 kPa (50 bis 10.000 psi) umfaßt.

2. Verfahren nach Anspruch 1, worin der Katalysator einen Katalysatorpromotor umfaßt.

3. Verfahren nach Anspruch 2, worin der Katalysatorpromotor eine säure umfaßt.

4. Verfahren nach Anspruch 3, worin das Verhältnis von Grammäquivalent Säure zu Grammatom Kobalt im Katalysator im Bereich von 0,001 bis 4:1, vorzugsweise von 0,01 bis 2:1 liegt.

5. Verfahren nach einem der Ansprüche 2 bis 4, worin der Katalysatorpromotor einen Metallsalzpromotor, ausgewählt aus einem Salz eines Metalles der Gruppe IA, Gruppe IIA, Gruppe IIB, Gruppe IIIB und der Seltenen Erdenreihe des Periodensystems der Elemente (CAS-Version), umfaßt.

6. Verfahren nach Anspruch 5, worin das Verhältnis von Grammäquivalent Metallsalz zu Grammatom Kobalt im Katalysator im Bereich von 0,001:1 bis 2:1, vorzugsweise von 0,01 bis 1:1, stärker bevorzugt von 0,1:1 bis 0,5:1 liegt.

7. Verfahren nach einem der Ansprüche 1 bis 4, worin zusätzlich ein Rutheniumkatalysator vorliegt.

8. Verfahren nach Anspruch 7, worin das Atomverhältnis Co:Ru im Bereich von 1.000:1 bis 1:100, vorzugsweise von 100:1 bis 1:10, stärker bevorzugt von 50:1 bis 1:5 liegt.

9. Verfahren nach einem der Ansprüche 1 bis 8, worin das Phosphin vor dem Komplexieren mit dem Kobaltcarbonylkatalysator partiell oxidiert wird, um ein Sauerstoff:Phosphor-Verhältnis im Bereich von 0,01:1 bis 0,5:1 auszubilden.

10. Verfahren nach Anspruch 9, worin das Phosphin partiell oxidiert wird, um ein Sauerstoff:Phosphor-Verhältnis im Bereich von 0,05:1 bis 0,3:1 auszubilden.

11. Verfahren nach einem der Ansprüche 1 bis 10, worin das 1,2-Epoxid aus 1,2-Epoxyalkanen oder 1,2-Epoxyalkenen mit 2 bis 30 Kohlenstoffatomen, vorzugsweise 2 bis 20, stärker bevorzugt 2 bis 10 Kohlenstoffatomen ausgewählt wird.

12. Verfahren nach Anspruch 11, worin das 1,2-Epoxid Ethylenoxid oder Propylenoxid, vorzugsweise Ethylenoxid ist.

13. Verfahren nach einem der Ansprüche 1 bis 12, worin das Phosphin ein α,Ω-Hydrocarbylen-P,P'-bis (monophosphabicyclononan) ist, in welchen Ringsystemen (a) jedes Phosphoratom ein Glied einer Brückenverknüpfung ist, (b) jedes Phosphoratom nicht in einer Brückenkopfposition vorliegt und (c) jedes Phosphoratom nicht ein Glied des bicyclischen Systems des anderen ist und (d) die kleinsten phosphorhältigen Ringe wenigstens 4, vorzugsweise wenigstens 5 Atome enthalten.

14. Verfahren nach einem der Ansprüche 1 bis 13, worin das Phosphin der Formel 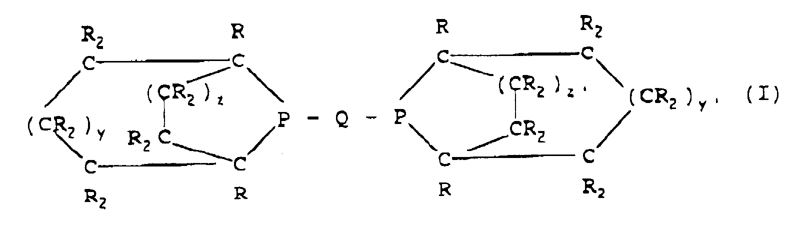 entspricht, worin Q für Hydrocarbylen mit bis zu 30 Kohlenstoffatomen steht; y und z Null oder positive ganze Zahlen darstellen deren Summe von 0 bis 7 ausmacht; y' und z', unabhängig von den Werten für y und z, für Null oder ganze Zahlen stehen, deren Summe 0 bis 7 beträgt; und R unabhängig für Wasserstoff oder Alkyl mit 1 bis 30 Kohlenstoffatomen steht.

15. Verfahren nach einem der Ansprüche 1 bis 14, wobei im Phosphin Q unter Ethylen, Propylen und Butylen ausgewählt ist.

16. Verfahren nach einem der Ansprüche 1 bis 15, worin in dem Phosphin das Monophosphabicyclononan unter 9-Phospha-bicyclo[4.2.1]nonan, 9-Phosphabicyclo [3.3.1]nonan und deren Gemischen ausgewählt ist.

17. Verfahren nach einem der Ansprüche 1 bis 16, worin das Phosphor:Kobalt-Atomverhältnis in dem Katalysator im Bereich von 0,1;1 bis 3:1, vorzugsweise 0,5:1 bis 2:1, stärker bevorzugt von 1:1 bis 1,5:1 liegt.

## Revendications

1. Procédé de préparation de 1,3-diols et de 3-hydroxyaldéhydes par l'hydroformylation de 1,2-époxydes, lequel procédé comprend la mise en contact intime
(a) d'un 1,2-époxyde comportant au moins 2 atomes de carbone,
(b) un catalyseur au cobalt carbonyle modifié par une phosphine ditertiaire, ladite phosphine comprenant un hydrocarbylène-bis-(monophosphabicycloalcane), dans lequel chaque atome de phosphore est lié au radical hydrocarbylène et est un élément d'une liaison de pontage sans être un atome tête de pont et lequel hydrocarbylène-bis(monophosphabicycloalcane) possède de 11 à 300 atomes de carbone, dont 5 à 12 atomes de carbone constituent, ensemble, avec un atome de phosphore, des membres de chacune des structures formant squelette bicyclique,
(c) du monoxyde de carbone, et
(d) de l'hydrogène, le rapport molaire du monoxyde de carbone à l'hydrogène variant de 4:1 à 1:6, de préférence, de 1:1 à 1:4,
en solution à phase liquide dans un solvant de réaction inerte, à une température de 30 à 150°C et sous une pression de 345 à 68948 kPa (50 à 10 000 psi).

2. Procédé suivant la revendication 1, caractérisé en ce que le catalyseur comprend un promoteur de catalyseur.

3. Procédé suivant la revendication 2, caractérisé en ce que le promoteur de catalyseur comprend un acide.

4. Procédé suivant la revendication 3, caractérisé en ce que le rapport des équivalents-grammes d'acide aux atomes-grammes de cobalt dans le catalyseur varie de 0,001 à 4:1, de préférence, de 0,01 à 2:1.

5. Procédé suivant l'une quelconque des revendications 2 à 4, caractérisé en ce que le promoteur de catalyseur comprend un promoteur à sel de métal choisi parmi un sel d'un métal du groupe IA, du groupe IIA, du groupe llB, du groupe IIIB et de la série des terres rares du tableau périodique des éléments (version CAS).

6. Procédé suivant la revendication 5, caractérisé en ce que le rapport des équivalents-grammes du sel de métal aux atomes-grammes du cobalt dans le catalyseur varie de 0,001:1 à 2:1, de préférence, de 0,01 à 1:1, plus avantageusement encore, de 0,1:1 à 0,5:1.

7. Procédé suivant l'une quelconque des revendications 1 à 4, caractérisé en ce qu'un catalyseur au ruthénium supplémentaire est présent.

8. Procédé suivant la revendication 7, caractérisé en ce que le rapport atomique Co:Ru varie de 1000:1 à 1:100, de préférence, de 100:1 à 1:10, plus avantageusement, de 50:1 à 1:5.

9. Procédé suivant l'une quelconque des revendications 1 à 8, caractérisé en ce que la phosphine, avant d'être complexée avec ledit catalyseur au cobaltcarbonyle est partiellement oxydée pour donner un rapport oxygène à phosphore qui varie de 0,01:1 à 0,5:1.

10. Procédé suivant la revendication 9, caractérisé en ce que la phosphine est partiellement oxydée pour donner un rapport oxygène à phosphore qui varie de 0,05:1 à 0,3:1.

11. Procédé suivant l'une quelconque des revendications 1 à 10, caractérisé en ce que l'on choisi le 1,2-époxyde parmi les 1,2-époxyalcanes et les 1,2-époxyalcènes, qui possèdent des nombres d'atomes de carbone qui fluctuent de 2 à 30, de préférence, de 2 à 20, plus avantageusement, de 2 à 10.

12. Procédé suivant la revendication 11, caractérisé en ce que le 1,2-époxyde est l'oxyde d'éthylène ou l'oxyde de propylène, de préférence, l'oxyde d'éthylène.

13. Procédé suivant l'une quelconque des revendications 1 à 12, caractérisé en ce que la phosphine est un α,Ω-hydrocarbylène-P,P'-bis(monophosphabicyclononane), dans lequel système cyclique (a) chaque atome de phosphore est un membre d'une liaison de pontage, (b) chaque atome de phosphore n'est pas dans une position tête de pont et (c) chaque atome de phosphore n'est pas un membre du système bicyclique de l'autre et (d) les noyaux contenant du phosphore les plus petits contiennent au moins quatre atomes, de préférence, au moins cinq atomes.

14. Procédé suivant l'une quelconque des revendications 1 à 13, caractérisé en ce que la phosphine répond à la formule suivante : 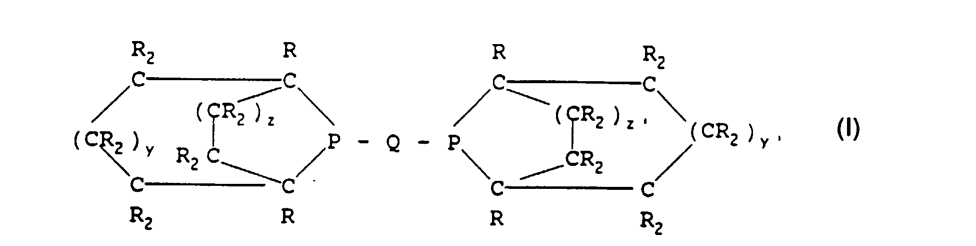 dans laquelle Q représente un radical hydrocarbylène comptant jusqu'à 30 atomes de carbone; y et z sont égaux à zéro ou représentent des nombres entiers positifs dont la somme varie de 0 à 7; y' et z', indépendamment des valeurs de y et de z, sont égaux à zéro ou représentent des nombres entiers positifs dont la somme varie de 0 à 7; et les symboles R représentent indépendamment des atomes d'hydrogène ou des radicaux alkyle possédant de 1 à 30 atomes de carbone.

15. Procédé suivant l'une quelconque des revendications 1 à 14, caractérisé en ce que, dans la phosphine, Q est choisi parmi l'éthylène, le propylène et le butylène.

16. Procédé suivant l'une quelconque des revendications 1 à 15, caractérisé en ce que, dans la phosphine, on choisit le monophosphabicyclononane parmi le 9-phosphabicydo[4.2.1]nonane, le 9-phosphabicyclo[3.3.1]nonane et leurs mélanges.

17. Procédé suivant l'une quelconque des revendications 1 à 16, caractérisé en ce que le rapport atomique phosphore à cobalt dans le catalyseur varie de 0,1:1 à 3:1, de préférence, de 0,5:1 à 2:1, plus avantageusement, de 1:1 à 1,5:1.
